# EUROPEAN PATENT SPECIFICATION

(11) **EP 0 957 899 B1**
(45) Date of publication and mention of the grant of the patent: **19.03.2003**
(21) Application number: 97936421.3
(22) Date of filing: 08.08.1997
(51) Int. Cl.: A61K 9/00

(54) **DOSAGE FORM FOR PROVIDING ASCENDING DOSE OF DRUG**
VERABREICHUNGSFORM ZUR ABGABE VON STEIGENDEN WIRKSTOFFDOSEN
FORME GALENIQUE DESTINEE A FOURNIR UNE DOSE CROISSANTE DE MEDICAMENT

(30) Priority: 16.08.1996 US 23286 P
(43) Date of publication of application: 24.11.1999
(73) Proprietor: ALZA Corporation, Mountain View, CA 94039-7210 (US)
(72) Inventor: SHIVANAND, Padmaja, Mountain View, CA 94040 (US); AYER, Atul, D., Palo Alto, CA 94303 (US); WRIGHT, Jeri, D., Dublin, CA 94568 (US); LAM, Andrew, San Francisco, CA 94122 (US); HAMEL, Lawrence, G., Mountain View, CA 94040 (US)
(74) Representative: Stebbing, Peter John Hunter
(86) International application number: US9713816
(87) International publication number: WO98006380

(56) References cited:
- EP-A- 0 621 032
- WO-A-95/20946
- FR-A- 2 598 319
- FR-A- 2 620 025
- GB-A- 2 206 046
- GB-A- 2 206 047
- US-A- 4 327 725
- US-A- 5 017 381

## Description

### DISCLOSURE OF TECHNICAL FIELD

This invention pertains to a dosage form for administering a drug in an ascending dose. The invention concerns further a dosage form comprising a drug in different concentrations for administering in a sustained ascending rate for therapy. The invention relates also to a laminate for use in a dosage form comprising a first lamina and a second lamina with each lamina comprising a first concentration and a second concentration of drug. The invention relates further to a method for achieving a therapeutic effect by administering a dosage form that administers an initial dose followed by a continuously increasing dose of drug over time.

### BACKGROUND OF THE INVENTION

For a long time, pharmacy and medicine in every society used medicines for pain relief, mood, thought, feeling, behavior, and psychological personality benefits. The medicines used for these therapies are represented by opioids, barbiturates, hypnotics, central nervous system stimulants, psychostimulants, alcohols, cannabinoids and catecholamines. While these medicines or drugs have a benefit, a serious problem, called tolerance, is associated with their use. The development of tolerance to a drug results from adaptive changes within the affected patient, such that the therapeutic response is reduced in the presence of the same concentration of drug. Tolerance to some drugs, for instance, to opioids, is characterized by a shortened duration and decreased intensity of the therapeutic effect. Most of the tolerance seen with many drugs is due to adaptation of cells in the nervous system to the drug's action, as noted in The Pharmacological Basis of Therapeutics by Goodman and Gilman, 7th Edition, p. 534 (1940).

In the present practice of medicine, one class of these drugs that has become the standard intervention for the management of behavior and personality, including attention deficit disorder, is the central nervous system stimulants. While this invention presents the central nervous system drug in detail, it is understood the invention is generic and it embraces drugs broadly as administered by the dosage form, and the mode and the manner of the invention.

The benefits perceived by physicians, psychologists, social workers and clinicians are dramatic for central nervous system drugs, and this has resulted in the widespread and acceptable use of central nervous system medication to treat attention deficit disorder. In the latest period for collecting data, it was observed that about two percent of the school-aged female population and about six percent of the school-aged male population, for a total of about two million patients, were administered medication for attention deficit disorder.

Prior to this invention, opioids, barbiturates, hypnotics, central nervous system stimulants, psychostimulants, alcohols, cannabinoids and catecholamines were administered by a standard pharmaceutical dosage form. For example, one prior art method for administering a drug consists in using an immediate release tablet containing the drug. This immediate release form delivers the drug by instant dumping of the drug and produces uneven blood levels characterized by peaks and valleys. For an immediate release dosage form containing a drug that has a rapid onset, and a short half-life, this drug may need multiple doses each day and this can result in swings in blood levels as the medication loses its therapeutic effect. This dosage form does not provide the needed therapy over an extended time.

Another prior art dosage form for dispensing a drug is the sustained-release dosage form. The sustained-release dosage form dispenses a drug in a non-ascending profile over time. This dosage form, however, may not provide the required therapy and the appropriate blood pattern. For drugs, such as the central-nervous system acting drugs, that are dispensed from a sustained-release non-ascending dose, the patient often develops an acute or a chronic tolerance to the drug manifested by a shortened duration and a decrease in the intensity needed for acceptable therapy. The prior art sustained-release dosage form is devoid of means that compensate for its shortcomings inherent therein.

FR-A-2620025 discloses a tablet comprising first and second layers comprising acetaminophen and hydrophillic polymers. The tablet is coated with a semi-permeable coating.

WO-A-9520946 discloses a tablet formulation for oral administration comprising first and second layers which respectively include amoxycillin and/or clavulanate, with the overall tablet comprising amoxycillin. The tablet provides for sustained release of amoxycillin, and optionally clavulanate, with the relative rate of release of amoxycillin and/or clavulanate from the first and second layers being different.

EP-A-0621032 discloses an osmotic delivery device for the oral administration of a pharmaceutically acceptable agent, which comprises a solid core containing an active agent, a delay jacket coated over the core, a semi-permeable membrane coated over the delay jacket, optionally having a release orifice, and an enteric coating over the semi-permeable membrane.

US-A-4327725 discloses an osmotic device which comprises a semi-permeable wall surrounding a compartment housing an agent that is insoluble to very soluble in aqueous and biological fluids, and a layer of fluid swellable hydrogel. A passageway in the wall connects the agent with the exterior of the device.

The above presentation teaches that a critical and timely need exists for a novel dosage form for administering a drug that overcomes the shortcomings known to the prior art. This long-felt need exists for a dosage form for (1) administering the drug in a sustained-increasing rate that simultaneously reduces or eliminates the frequency of daily dosing; for (2) administering a drug in a sustained-compensating dose to substantially compensate for acute tolerance to the drug and thereby maintaining a pre-selected clinical response; for (3) administering the drug in an increasing dose to lessen or eliminate chronic tolerance to the drug to provide effective therapy; and for (4) administering the drug in a sustained-ascending profile clinically indicated for both medical and psycho-medical effects.

### OBJECTS OF THE INVENTION

Accordingly, in view of the above presentation, it is an immediate object of this invention to provide a novel and unique dosage form that provides an ascending dose of drug over time.

Another object of the invention is to provide a dosage form that delivers a drug in a controlled increasing dose over an extended time to maintain a therapeutic effect.

Another object of the invention is to provide a dosage form for maintaining the therapeutic effect of a drug in a patient that acquires tolerance to the drug, wherein the dosage form delivers to the patient the drug in a controlled increasing dose over time.

Another object of the invention is to provide a dosage form for maintaining the therapeutic effect of a drug in a patient that acquires acute tolerance to the drug, wherein the dosage form delivers to the patient the drug in a controlled increasing dose to provide compensation for the acquired acute tolerance associated with the drug.

Another object of the invention is to provide a dosage form for maintaining the therapeutic effect of a drug in a patient that acquires chronic tolerance to the drug, wherein the dosage form delivers to the patient the drug in a controlled increasing dose to provide compensation for the acquired chronic tolerance associated with the drug.

Another object of the invention is to make available a dosage form for lessening the incidence of acquired tolerance in a patient administered a drug that develops tolerance in the patient, wherein the dosage form is characterized by administering the drug in a sustained and increasing dose over time to produce the intended effect.

Another object of the invention is to provide a dosage form for administering an opioid, barbiturate, hypnotic, central nervous system stimulant, psychostimulant, alcohol, cannabinoid or catecholamine drug that overcomes the shortcomings known to the prior art.

Another object of the invention is to provide a dosage form comprising means for compensating for tolerance development associated with a drug possessing the ability to produce tolerance in a patient, by the dosage form administering the drug orally in a sustained-ascending dose to substantially lessen the unwanted effects of tolerance.

Another object of the invention is to make available a method for lessening the incidence of acquired tolerance in a patient administered a drug that develops tolerance in the patient, wherein the method comprises administering a dosage form that dispenses a drug in a sustained and increasing dose over time to produce the intended effect.

Another object of the invention is make available a method for administering a drug in a sustained and increasing dose by administering a dosage form that delivers the drug in a sustained and increasing dose over time.

Another object of the invention is to provide a dosage form comprising a first layer comprising a first concentration of drug and a second layer comprising a second and higher concentration of drug that are delivered first then second to provide a sustained and ascending delivery of drug.

Another object of the invention is to provide a method for administering a drug in a sustained and increasing dose by administering a dosage form comprising a first concentration of drug and a second higher concentration of drug which dosage form delivers the first followed by the second concentration of drug to give a sustained and ascending delivery of drug.

Another object of the invention is to provide a dosage form that administers a drug in a sustained and ascending pattern over time, which dosage form is characterized by a first drug layer comprising a lower concentration of drug compared to a second drug layer that are delivered consecutively to provide the ascending pattern.

Another object of the invention is to provide a pharmaceutical, oral tablet manufactured as a solid dosage form comrpising a first layer containing 10 ng to 300 mg of drug and a pharmaceutically acceptable carrier, and a second layer containing 50 ng to 500 mg of drug and a pharmaceutically acceptable carrier, which first and second layers release drug in a serial order for administering a sustained and increasing dose of drug.

Another object of the invention is to provide a pharmaceutical, osmotic tablet manufacted as an osmotic dosage form comprising a first layer containing 10 ng to 300 mg of drug and a pharmaceutically acceptable carrier, a second layer containing 50 ng to 500 mg of drug and a pharmaceutically acceptable carrier, and a third layer for displacing the first and second layers in succession from the dosage form, for administering a sustained and increasing dose of drug.

Another object of the invention is to provide an osmotic dosage form comprising a first layer comprising a first dose of drug, a second layer comprising a layer dose of drug than the first layer, a third layer for pushing the first layer then the second layer from the dosage form, and a semipermeable wall with an exit that surrounds the layer for delivering an increasing dose of drug over sixteen hours, or longer.

Another object of the invention is to provide a bilayer arrangement comprising a first drug layer comprising a lower concentration of drug compared to a second drug layer.

Another object of the invention is to provide a trilayer comprising a first drug layer comprising a lower concentration of drug layer compared to a second drug layer, and a third layer that provides physical support to the first and second layers for delivery of the first and second layers to an environment of use.

Another object of the invention is to provide a composition of matter comprising: (1) a drug selected from the groups consisting of opioid, barbiturate, hypnotic, central nervous system stimulant, psychostimulant, alcohol, cannabinoid, and catecholamine; (2) a pharmaceutically acceptable hydrophilic polymeric carrier; and (3) a hydrophobic compound that aids in controlling the rate of hydration of the composition.

Another object of the invention is to provide an osmotic dosage form comprising an immediate dose of drug on the exterior of the dosage form, and a prolonged ascending dose in the interior of the dosage form, which two doses operate in combination to provide an ascending dose of drug to negate acquired and developed tolerance.

Another object of the invention is to provide a tablet for oral use comprising a first layer comprising a first concentration of drug and a pharmaceutically acceptable carrier and a second layer comprising a second concentration of drug and a pharmaceutically acceptable carrier, and wherein the tablet comprises a larger dose of drug in the second layer.

These objects, as well as other objects, features and advantages of this invention will become more apparent from the following detailed disclosure of the invention accompanied by the accompanying claims.

### DESCRIPTION OF DRAWING FIGURES

Figure 1 depicts the ascending release rate for the drug methylphenidate from a dosage form provided by the invention.
Figure 2 depicts the ascending release rate for the drug pseudoephedrine from a dosage form provided by the invention.

### DETAILED DESCRIPTION OF SPECIFICATION

In accordance with the practice of this invention, it has now been discovered that the invention can make available a dosage form with an ascending rate of drug delivery over time. The dosage form of this invention comprises a wall that surrounds an internal compartment. The wall of the dosage form is permeable to the passage of fluid present in an environment of use, such as the aqueous-biological fluid of the gastrointestinal tract, and the wall is impermeable to the passage of drug. The wall comprises a semipermeable composition that maintains its physical and chemical integrity during the drug dispensing life of the dosage form. The semipermeable wall comprises a polymer selected from the group consisting of a cellulose acylate, cellulose diacylate, cellulose triacylate, cellulose acetate, cellulose diacetate and cellulose triacetate. The wall can comprise 65 wt% (weight percent) to 100 wt% of said polymer. The wall can comprise 0 to 35 wt% polyethylene glycol possessing a number average molecular weight of 190 to 20,000, from 0 to 35 wt% of a hydroxypropylalkylcellulose selected from the group consisting of hydroxypropylmethylcellulose, hydroxypropylethyicellulose, hydroxypropylbutylcellulose, and hydroxypropylpentylcellulose of number average molecular weight 9,000 to 250,000, and 0 to 35 wt% of a hydroxyalkylcellulose including a member selected from the group consisting of hydroxymethylcellulose, hydroxyethylcellulose, hydroxypropylcellulose and hydroxybutylcellulose of number average molecular weight 7,500 to 200,000. The total weight of all components comprising the wall is equal to 100 wt%. Wall-forming polymers are known in U.S. Patent Numbers 3,845,770; 3,916,899; 4,036,228; 4,111,202; and 5,178,866.

The dosage form comprises at least one exit in the wall that connects the exterior of the dosage form with the interior of the dosage form. The expression "exit" as used herein comprises a passageway, orifice, bore, pore, micropore, hollow fiber, capillary tube, porous overlay, porous insert and the like for dispensing a drug from the dosage form. The exit passageway includes a material that erodes, or is leached from the wall in a fluid environment of use, such as the gastrointestinal tract. Representative materials for forming a passageway, or more than one passageway, include erodible poly(glycolic) acid, erodible poly(lactic acid), erodible poly(orthoester), erodible poly(orthocarbonate), a gelatinous filament, poly(vinyl alcohol), leachable materials including fluid removable pore-forming polysaccharides, salts and oxides. An exit can be formed by leaching compounds such as sorbitol, lactose or glucose. The exit can have any shape such as round, triangular, square or elliptical. The dosage form can be provided with one or more passageways in spaced apart positions on a common surface of the dosage form Passageways and equipment for forming an exit are disclosed in U.S. Patent Numbers 3,845,770; 3,916,899; 4,063,064; 4,088,864; 4,200,098; 4,285,987; and 5,178,866.

The dosage form's compartment comprises a first layer comprising a dose of drug and a second layer comprising a larger dose of drug. The first layer and the second layers are delivered in successive order. The first layer is next to the exit to provide for its delivery first, followed by the second layer, from the dosage form to give an ascending profile of delivered drug. The first layer comprises 10 ng (nanograms) to 250 mg (milligrams) of drug and a pharmaceutically acceptable carrier; and the second layer comprises 50 ng to 500 mg of drug and a pharmaceutically acceptable carrier. The administration of the first dose followed by the second dose provides a controlled-increasing dose that simultaneously reduces or eliminates the frequency of daily dosing and compensates for tolerance to the drug. The first layer delivers its dose over a period of 30 minutes to 5 hours and the second layer delivers its dose over 1.5 hours to 9 hours.

The first layer provided by the invention comprises: (a) a dose of 10 ng to 250 mg of drug; (b) 5 mg to 250 mg of a pharmaceutically acceptable carrier comprising hydrophilic polymer comprising a poly(alkylene oxide) of number average molecular weight 40,000 to 1,000,000, with a 5% aqueous solution exhibiting a viscosity at 25°C of 12 to 17,600 cps (centipoise) represented by a member selected from the group consisting of poly(methylene oxide), poly(ethylene oxide), poly(propylene oxide), poly(butylene oxide), copolymer poly(ethylene oxide)-(polypropylene oxide), and a blend of two different poly(alkylene oxides) such as poly(ethylene oxide) of number average molecular weight 100,000 and a blend of two different pharmaceutically acceptable carriers comprising (polyethylene oxide) of 200,000 number average molecular weights, and poly(ethylene oxide) of 200,000-(polyethylene oxide) of 300,000 number average molecular weights, which poly(alkylene oxide) polymers are available from Union Carbide Corporation; 0 mg to 100 mg of a hydrophilic pharmaceutically acceptable carboxyvinylpolymer known also as carboxypolyalkylene polymers possessing a number average molecular weight of 7,500 to 3,000,000, including a carboxyvinylpolymer of number average molecular weight 450,000, a carboxyvinylpolymer of number average molecular weight 750,000, a carboxyvinylpolymer of number average molecular weight 1,250,000 and a carboxyvinylpolymer of number average molecular weight 3,000,000, as disclosed in U.S. Patent Numbers 2,798,053, 2,909,462 and 3,825,068 and available as Carbopol® polymer from the B. F. Goodrich Company; and 0 mg to 250 mg of a pharmaceutically acceptable alkali carboxymethylcellulose wherein the alkali is sodium or potassium represented by sodium carboxymethylcellulose of viscosity average number molecular weight 10,000 to 700,000, available from the Hercules Corporation, with the expression "pharmaceutically acceptable" meaning nontoxic and acceptable for oral administration to a human patient; (c) a surfactant selected from 0.05 mg to 7.5 mg of a member selected from the group consisting of amphoteric, anionic, cationic, and nonionic surfactants, as represented by sorbitan trioleate, sorbitan tristearate, ethylene glycol fatty acid ester, polyethylene glycol monostearate, sorbitan sesquioleate, glycerol monostearate, sorbitan mono-oleate, propylene glycol monolaurate, sorbitan monostearate, diethylene glycol monolaurate, sorbitan monopalmitate, polyoxyethylene mannitol dioleate, sorbitan monolaurate, polyoxyethylene lauryl ether, polyoxyethylene monostearate, polyethylene glycol 400 monostearate, triethanolamine oleate, polyoxyethylene alkyl phenol, polyethylene alkyl aryl ether, polyoxyethylene sorbitan monolaurate, polyoxyethylene sorbitan monostearate, polyoxyethylene sorbitan monooleate, polyoxyethylene monostearate, polyoxyethylene sorbitan monopalmitate, polyoxyethylene monostearate, polyoxyethylene sorbitan monolaurate, polyoxyethylene lauryl ether, polyoxyethylene monostearate, sodium oleate, and sodium lauryl sulfate, which surfactants are known in Pharmaceutical Sciences by Remington, 17th Edition, pp. 1305-1306 (1985); (d) 0.5 mg to 20 mg of a hydroxypropylalkylcellulose binder polymer comprising a member selected from the group consisting of hydroxypropylmethylcellulose, hydroxypropylethylcellulose, hydroxypropylbutylcellulose and hydroxypropylpentylcellulose of number average molecular weight 9,000 to 750,000, available from the Dow Chemical Company; and 0.0 mg to 20 mg of a hydroxyalkylcellulose selected from the group consisting of hydroxymethylcellulose, hydroxyethylcellulose, hydroxypropylcellulose, hydroxybutylcellulose and hydroxypentylcellulose of number average molecular weight 7,500 to 750,000, available from Aqualon Company; and (e) 0.01 mg to 5 mg of a lubricant such as stearic acid, magnesium stearate, calcium stearate, potassium oleate, magnesium laureate or calcium palmitate.

The second layer in contacting arrangement with the first layer comprises 50 ng to 500 mg of drug, and to obtain an ascending release profile of delivered drug, the drug concentration in the second layer is larger than the drug concentration in the first layer. The second layer comprises: (a) a dose of 50 ng to 500 mg of drug; (b) 25 mg to 450 mg of a pharmaceutically acceptable carrier illustrated by hydrophilic hydrogel polymer such as poly(alkylene oxide) of number average molecular weight 40,000 to 1,000,000 mg; 0 mg to 100 mg of a carboxyvinylpolymer of number average molecular weight 7,500 to 3,000,000; and 0 ng to 250 mg of an alkali carboxymethylcellulose of viscosity average molecular weight 10,000 to 700,000; (c) 0.05 mg to 7.5 mg of a surfactant; (d) 0.5 mg to 20 mg of a hydroxypropylalkylcellulose of number average molecular weight 9,000 to 750,000, and 0.0 mg to 20 mg of a hydroxyalkylcellulose of number average molecular weight 7,500 to 750,000; and (e) 0.01 mg to 5 mg of a lubricant. The polymers, surfactants, and the lubricants are disclosed with the first layer.

The dosage form comprises a third layer that displaces or pushes the first and second layers consecutively through the exit port from the dosage form. The second layer comprises: (a) 15 ng to 450 mg of a hydrophilic osmopolymer selected from the group consisting of a poly(alkylene oxide) of number average molecular weight 2,000,000 to 10,000,000, or an alkali carboxymethylcellulose of viscosity average molecular weight 2,000,000 to 10,000,000; (b) 2 mg to 50 mg of an osmagent, also known as osmotically effective solute, osmotically effective compound, and osmotic agent including inorganic and organic compounds represented by an osmagent selected from the group consisting of magnesium sulfate, magnesium chloride, sodium chloride, lithium chloride, potassium sulfate, sodium sulfate, lithium sulfate, potassium chloride, sodium sulfate, magnesium succinate, tartaric acid, carbohydrate, raffinose, sucrose, glucose, and lactose; (c) 0.01 mg to 10.0 mg of a surfactant including amphoteric, anionic, cationic and nonionic surfactants as presented under the first layer; (d) 0 mg to 20 mg of a carboxyvinylpolymer of number average molecular weight 7,500 to 10,000,000 as presented under the first layer; (e) 0.1 mg to 30 mg of a hydroxypropylalkylcellulose of number average molecular weight 9,200 to 750,000, including the hydroxypropylalkylcellulose polymer presented under the first layer; (f) 0.0 mg to 5 mg of a colorant compound to identify the dosage form, such as red ferric oxide; and (g) 0.0. mg to 5 mg of a lubricant, including the lubricants presented under the first layer.

The drug present in the first and second layer comprises opioids, barbiturates, hypnotics, central nervous system acting drugs, psychostimulants, alcohols, cannabinoids and catecholamines. Examples of drug are the central nervous system acting drugs useful for the management of attention deficit disorder, including catecholamines and drugs that can mimic their action. The drugs useful for this therapy comprise a member selected from the group consisting of amphetamine, dextroamphetamine, methamphetamine, methylphenidate, racemic methylphenidate, theomethylphenidate, ethylphenidate, phenylisopropylamine and pemoline. The drugs include also their pharmaceutically acceptable salts such as a member selected from the group consisting of hydrochloride, sulfate, phosphate, acetate, hydrobromide, pamoate and maleate.

The invention comprises further a coat on the external surface of the dosage form. The coat is an external overcoat carried by the dosage form. The external overcoat on the wall of the dosage form comprises a dose of drug, and the overcoat cooperates with the interior compartment comprising a dose of drug that delivers the drug to provide an unexpected ascending drug delivery profile. The overcoat provides an initial dose of drug followed by a larger dose of drug from the interior of the dosage form to give an ascending drug delivery profile. The overcoat comprises 100 ng to 100 mg of a drug that is delivered in up to 2 hours followed by the larger dose from the dosage form. The overcoat comprises a drug selected from the group consisting of opioids, barbiturates, hypnotics, psychostimulants, central nervous system acting drugs and catecholamines. Representative of individual drugs present in the overcoat comprise a drug selected from the group consisting of amphetamine, dextro-amphetamine, methamphetamine, methylphenidate, racemic methylphenidate, ethylphenidate, alkylphenidate, phenylisopropylamine, and pemoline. These drugs include also their pharmaceutically acceptable salts such as a member selected from the group consisting of hydrochloride, sulfate, phosphate, acetate, hydrobromide, pamoate and maleate. Representative of a drug embodiment present in the overcoat is alkylphenidate comprising 10 ng to 20 mg of methylphenidate.

The overcoat comprises the drug blended with a pharmaceutically acceptable carrier consisting of an aqueous-releasing carrier, alkylcellulose, methylcellulose, ethylcellulose, hydroxyalkylcellulose, hydroxymethylcellulose, hydroxypropylcellulose, hydroxyethylcellulose, hydroxybutylcellulose, hydroxypropylalkylcellulose, hydroxypropylmethylcellulose, hydroxypropylethylcelllulose, hydroxypropylbutylcelllulose, methyldextrose, acacia guar gum, pregelatinized starch, propylene glycol alginate and cyclodextrin. The overcoat comprises 0 to 5 wt%, in one operative embodiment 0.01 to 5 wt% of polyethylene glycol, polypropylene glycol, polyvinyipyrrolidone, and acetylated triglyceride. The overcoat provides needed drug therapy, for example methylphenidate, as the overcoat dissolves or undergoes dissolution in the presence of fluid present in the gastrointestinal tract of a patient. Thus, the overcoat provides drug therapy on oral administration into the patient's drug receiving environment, the gastrointestinal tract.

The wall of the dosage form in one manufacture is formed by an air suspension procedure. This procedure consists of suspending and tumbling the compressed trilaminate in a current of air and wall-forming composition until a wall is applied forming an internal compartment containing the trilayer core. The air-suspension procedure is well-suited for independently forming the wall. The air suspension procedure is described in U.S. Patent Numbers 2,799,241 and 5,082,668. The wall can be formed in an air suspension coater using cosolvents like methylenedichloride-methanol, 80:20 (wt:wt), or acetone-water cosolvent 85:15, 90:10 or 95:5, (wt:wt) using 2.5 to 5% solids. Other wall forming techniques can be used, such as a pan coating system, or a wall forming composition deposit by successive spraying of the composition accompanied by tumbling in a rotating pan. A pan coater is used to produce thicker walls. A large volume of solvent such as methanol, can be used in a cosolvent system to produce a thinner wall. Finally, the wall coated compartment is dried in an oven at 30°C to 50°C for up to a week, or in a humidity controlled oven at 50 RH (relative humidity) and 50°C for 18 hours to 3 days.

The first and second layers of the invention are made by standard manufacturing techniques. For example, in one manufacture the drug and other ingredients are blended and pressed into a solid layer. The drug and the ingredients can be blended with a solvent and mixed into a semisolid or solid formed by conventional methods such as ball-milling, calendering, stirring or roller milling and then pressed into a pre-selected shape. The layer possesses dimensions that correspond to the internal dimensions of the area the layer occupies in the dosage form, and it also possesses dimensions corresponding to the second and third layer for forming a contacting arrangement therewith. The dispensing or push layer comprising the osmopolymer is placed in contact with the second drug layer. The displacement layer is manufactured using the techniques for providing the first and second drug layers. The layering of the first drug layer, the second drug layer, and the displacement layer can be fabricated by conventional press-layering techniques. The three-layered compartment-forming core is surrounded and coated with an outer semipermeable wall. An exit is laser drilled through the wall to contact the first drug layer, with the dosage form optically oriented automatically by the laser equipment for forming the exit passageway.

In another manufacture, the dosage form is manufactured by the wet granulation technique. In the wet granulation technique, for example, the drug and the ingredients comprising a drug layer are blended using an organic solvent, such as isopropyl alcohol-methylene dichloride 80:20 (v:v) (volume:volume) or methanol-methylene dichloride, as the granulation fluid. Other granulating fluid such as denatured alcohol 100% can be used for this purpose. The ingredients forming the drug layer are individually passed through a screen and then thoroughly blended in a mixer. Next, other ingredients comprising the drug layer are dissolved in a portion of the granulation fluid, such as the cosolvent described above. Then, the latter prepared wet blend is slowly added to the drug blend with continual mixing in the blender. The granulating fluid is added until a wet blend is produced, which wet mass then is forced through a screen onto oven trays. The blend is dried for 18 to 24 hours at 30°C to 50°C. The dry granules are sized then with a screen. Next, a lubricant is passed through a screen and added to the dry screen granule blend. The granulation is put into milling jars and mixed on a jar mill for 1 to 15 minutes. The other drug layer and the displacement layers are made by the same wet granulation techniques. The compositions are pressed into their individual layers in a layer press.

Another manufacturing process that can be used for providing the compartment-forming composition layers comprises blending the powdered ingredients for each layer independently in a fluid bed granulator. After the powdered ingredients are dry blended in the granulator, a granulating fluid, for example, poly(vinyl-pyrrolidone) in water, or poly(vinyl-pyrrolidone) in denatured alcohol, or poly(vinyl-pyrrolidone) in 95:5 ethyl alcohol/water, or poly(vinyl-pyrrolidone) in blends of ethanol and water, is sprayed onto the powders. Optionally, the ingredients can be dissolved or suspended in the granulating fluid. The coated powders are then dried in a granulator. This process granulates all the ingredients present therein while adding the granulating fluid. After the granules are dried, a lubricant such as stearic acid or magnesium stearate is added to the granulator. The granules for each separate layer are then pressed in the manner described above.

The dosage form of the invention is manufactured in another manufacture by mixing a known dose of drug with compositional layer-forming ingredients and then pressing the composition into a solid layer possessing dimensions corresponding to the internal dimensions of the compartment. A contacting additional layer containing an increased dose of drug is manufactured in a like manner. In another manufacture a first and second layers are independently manufactured by mixing for each layer, the drug, other drug composition forming ingredients, and a solvent, to form a semisolid or solid by conventional laboratory methods such as ball milling, calendering, stirring or roll milling, and then each composition is pressed into a layer. Next, the first and different second layers and placed next to a layer of a displacement composition comprising an osmopolymer and an optional osmagent. Then, the three layered core is surrounded with a semipermeable wall. The layering of the first layer, the second, middle layer and the third layer can be accomplished by conventional layer tablet press techniques. The wall can be applied by molding, spraying, or dipping the pressed-shape trilayered-core into wall forming materials. Another technique that can be used for applying the wall is the air suspension coating procedure. This procedure consists in suspending and tumbling the three layered laminate in a current of air until the wall forming composition surrounds the trilayer. The air suspension procedure is described in U.S. Patent Number 2,799,241; *J. Am. Pharm. Assoc*., Vol. 48, pp. 451-459 (1979); and ibid., Vol. 49, pp. 83-84 (1960). Other manufacture procedures are described in Modern Plastic Encyclopedia, Vol. 46, pp. 62-70 (1969); and in Pharmaceutical Sciences by Remington, 14th edition, pp. 1626-1979 (1970), published by Mack Publishing Co., Easton PA.

Exemplary solvents suitable for manufacturing the wall, the first layer, and the second layer include inert inorganic and organic solvents. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane ethylene glycol monoethyl ether, ethylene glycol monoethyl acetate, methylene dichloride, ethylene dichloride, propylene dichloride, carbon tetrachloride, chloroform, nitroethane, nitropropane, tetrachloroethane, ethyl ether, isopropyl ether, cyclohexane, cyclo-octane, benzene, toluene, naphtha, tetrahydrofuran, diglyme, aqueous and nonaqueous mixtures thereof, such as acetone and water, acetone and methanol, acetone and ethyl alcohol, methylene dichloride and methanol, ethylene dichloride and methanol, and methylene dichloride and ethanol. The solvents are disclosed in U.S. Patent Number 5,030,456.

### DETAILED DESCRIPTION OF THE EXAMPLES

The following examples are illustrative of the present invention.

### EXAMPLE 1

A dosage form, designed and adapted to deliver a drug in an ascending release rate profile is manufactured according to this example.

First, a first layer forming composition comprising a dose of drug is manufactured as follows. 157.8 g of poly(ethylene oxide) having a number average molecular weight of 200,000 is passed through a 40 mesh screen, U.S. sieve, and placed into the bowl of a conventional planetary mixer. Next, 31.2 g of the drug methylphenidate hydrochloride is added to the mixer. Then, 10 g of hydroxypropylmethylcellulose of number average molecular weight 9,200 is passed through a 40 mesh sieve and added to the mixer comprising the methylphenidate hydrochloride and the poly(ethylene oxide). Then, 0.5 g of FD&C Blue Dye No. 1, for color identification, is added to the bowl of the mixer. The ingredients are blended in the mixer for 10 minutes to produce a homogenous composition. Next, about 100 ml of denatured anhydrous ethanol is added gradually to the mixer with continual mixing over a period of 5 to 10 minutes to change the consistency of the dry ingredients to wet granules. The wet granulation is passed through a 20 mesh screen, dried at room temperature for 16 hours, and then passed through a 20 mesh screen. Then, 0.5 g of magnesium stearate is passed through a 40 mesh screen, added to the homogenous composition and all the ingredients mixed for an additional minute.

Next, a second composition for providing a second layer is prepared as follows. First, 112.6 g of poly(ethylene oxide) of number average molecular weight 200,000 is passed through a 40 mesh screen and placed into the bowl of a conventional planetary mixer. Next, 76.4 g of the drug methylphenidate hydrochloride is added to the bowl containing the poly(ethylene oxide). Then, 10 g of hydroxypropylmethylcellulose of weight average molecular weight 9,200 is passed through a 40 mesh screen and added to the bowl containing the poly(ethylene oxide) and the methylphenidate hydrochloride. Then, 0.5 g of FD&C blue dye No. 1, or a different FD&C color dye to provide a color differential between the layers, is added to the mixer. All the ingredients are blended for 10 minutes to produce a homogenous blend. Next, 100 ml of denatured anhydrous ethanol is added to the mixer with continued mixing over a period of 5 to 10 minutes to change the dry powder blend into wet granules. The wet granulation is passed through a 20 mesh screen, dried at a room temperature of 72°F (22.2°C) for 16 hours and then passed though a 20 mesh screen. Next, 0.5 g of magnesium stearate is passed through a 40 mesh screen, added to the granulation, and all the ingredients mixed for an additional 1 minute.

Next, the displacement layer, also characterized as a third layer, is prepared as follows. First, 107 g of poly(ethylene oxide) of number average molecular weight 7,000,000, 80 g of osmagent sodium chloride, 10 g of hydroxypropylmethylcellulose of number average molecular weight 9,200, and 2 g of red ferric oxide are passed through a 40 mesh screen and then placed into the bowl of a mixer. The ingredients are blended together to form a homogenous blend. Next, 50 ml of denatured anhydrous ethanol is added to the mixer accompanied by continual mixing for 7 to 10 minutes, to produce wet granules. The wet granules are passed through a 20 mesh screen, dried at room temperature for 16 hours, and then passed through a 20 mesh screen. Next, 1 g of magnesium stearate is passed through a 40 mesh screen, added to the granulation, and all the ingredients mixed for an additional minute.

Next, the first composition and the second composition are pressed together into contacting layers, as follows. First, 33 mg of the first composition is added to a 7/32" (0.55 cm) die cavity and tamped lightly. Next, 24 mg of the second composition is added to the die and tamped lightly to provide the two drug layers. Then, 57 mg of the displacement composition is added to the die and the three layers are compressed together using 1/2 ton of pressure to form a trilayer tablet. The trilayer tablet weighs 114 mg.

Next, the trilayer is surrounded with a semipermeable wall as follows. First, a semipermeable wall forming composition is prepared comprising 95% cellulose having an acetyl content of 39.8% and 5% polyethylene glycol of number average molecular weight 3350, provided by Union Carbide Co., by dissolving the ingredients in a mixture of acetone and water in a 90:10 (wt:wt) ratio, to provide a solid composition at 5%. The trilayer tablets are placed in a pan coater and 15 mg of the semipermeable wall forming composition is sprayed onto the trilayer tablet. Next, a 30 mil (.76 mm) orifice is drilled on the drug side to connect the first layer with the outside of the dosage form. The dosage form tablets are dried at 50°C and 50% relative humidity to remove the residual solvents.

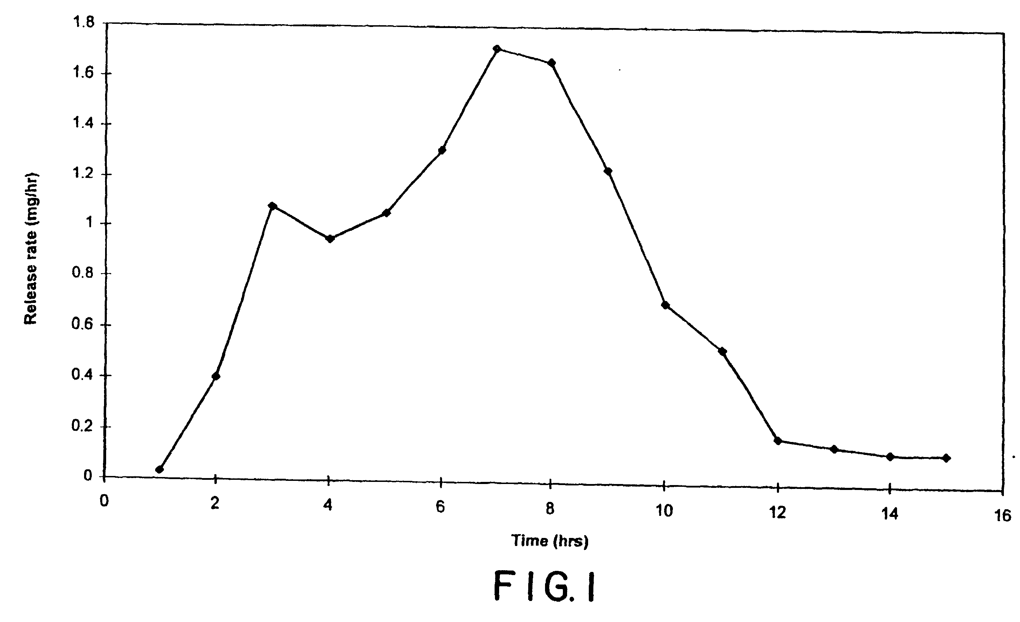

The dosage form comprises 5.2 mg of methylphenidate hydrochloride, 26 mg of poly(ethylene oxide) of number average molecular weight 200,000, 1.65 mg of hydroxypropylmethylcellulose of number average molecular weight 9,200, 0.083 mg of magnesium stearate, and 0.083 mg of dye in the first drug layer. The dosage form comprises 9.17 mg of methylphenidate hydrochloride, 13.52 mg of poly(ethylene oxide) of number average molecular weight 200,000, 1.2 mg of hydroxypropylmethylcellulose of number average molecular weight 9,200, 0.06 mg of magnesium stearate, and 0.06 mg of dye in the second drug layer. The third displacement layer comprises 30.5 mg of poly(ethylene oxide) of number average molecular weight 7,000,000, 22.8 mg of osmagent sodium chloride, 2.85 mg of hydroxypropylmethylcellulose of number average molecular weight 9,200, 0.57 mg of ferric oxide, and 0.285 mg of lubricant magnesium stearate. The semipermeable wall comprises 14.25 mg of cellulose acetate of 39.8 acetyl content, and 0.75 mg of polyethylene glycol of number average molecular weight 3350. The dosage form exhibited the following ascending release-rate profile comprising 0.03 mg in 1 hour, 0.4 mg in 2 hours, 1.08 mg in 3 hours, 0.95 mg in 4 hours, 1.06 mg in 5 hours, 1.31 mg in 6 hours, and 1.72 mg in 7 hours as further seen in accompanying Figure 1.

### EXAMPLE 2

The procedure in Example 1 is followed in this example, with the manufacture as described, but in this example the drug in the first layer and in the second layer are selected from the group of drugs consisting of amphetamine, dextro-amphetamine, methamphetamine, ethylphenidate, phenylisopropylamine and pemoline, with the dose of drug in the second layer larger than the dose of the same drug in the first layer, wherein the dose of drug in the first layer comprising 10 ng to 300 mg of drug and the dose of drug in the second layer comprising 50 mg to 500 mg of drug.

### EXAMPLE 3

A dosage form designed and adapted to deliver pseudoephedrine hydrochloride in an ascending release-rate profile over time is prepared according to this example.

A first drug layer forming composition is prepared by passing 139 g of poly(ethylene oxide) of number average molecular weight 300,000 through a 40 mesh screen and then placing the screened hydrophilic polymer into the bowl of a mixer. Next, 40 g of pseudoephedrine hydrochloride is added to the bowl and the ingredients blended for 5 minutes. Then, 10 g of hydroxypropylmethylcellulose of number average molecular weight 9,200 and 10 g of polyoxyethylene 40 stearate are screened through a 40 mesh screen and added to the bowl containing the previously added ingredients. Then, all the ingredients are blended together for 10 minutes to yield a homogenous composition. Next, 100 ml of denatured anhydrous ethanol is added to the mixer with continuous mixing over a period of 5 to 10 minutes to change the drug ingredients to a wet granulation. The wet granulation is passed through a 20 mesh screen, dried at room temperature for 16 hours, and then rescreened through a 20 mesh screen. Next, 1 g of magnesium stearate is screened through a 40 mesh screen and added to the mixer, and all the ingredients are mixed again for an additional minute.

Next, a second drug layer is prepared by passing 28.8 g of poly(ethylene oxide) of number average molecular weight 300,000 through a 40 mesh screen and then adding the screened hydrophilic polymer to a mixer. Next, 150 g of pseudoephedrine hydrochloride is added to the mixer and the ingredients blended for 3 minutes. Then, 10 g of hydroxypropylmethylcellulose of number average molecular weight 9,200, 10 g of polyoxyethylene 40 stearate, and 0.2 g of red ferric oxide are passed through 40 mesh screen and added to the mixer. Then, all the ingredients are blended for 10 minutes to produce a homogenous composition. Then, 100 ml of denatured anhydrous ethanol is added to the mixer and the mixing continued for 10 minutes to produce a wet granulation. Next, the wet granulation is passed through a 20 mesh screen, dried at room temperature for 16 hours, and then screened through a 20 mesh screen. Then, 1 g of magnesium stearate is passed through a 40 mesh screen, added to the granulation, and all the ingredients blended an additional minute.

Next, the displacement, or third drug-free layer is prepared as follows. First, 111 g of poly(ethylene oxide) having a number average molecular weight of 7,000,000, 60 g of sodium chloride, 10 g of hydroxypropylmethylcellulose having a number average molecular weight of 9,200, 10 g of polyoxyethylene 40 stearate, 6 g of cross-linked acrylic acid polymer of number average molecular weight 3,000,000, and 2 g of red ferric oxide are passed through a 40 mesh screen and added to the mixer. The ingredients are blended until a homogenous blend is formed. Then, 25 ml of denatured anhydrous ethanol is added to the mixer and mixing continued for 10 minutes to provide wet granules. The wet granules are passed through a 20 mesh screen, dried at room temperature for 16 hours, and then passed through a 20 mesh screen. Next, 1 g of magnesium stearate is passed through a 40 mesh screen, added to the granulation, and all the ingredients blended for an additional minute.

Next, the three layers are pressed into a three layered core. First, 22 mg of the first drug composition is added to the die and tamped lightly. Next, 18 mg of the second drug composition is added to the die and the second composition tamped lightly. Then, 40 g of the third displacement drug-free composition is added to the die, and the three layers compressed under 1/2 ton of pressure to form a trilayer tablet.

Next, the trilayer is surrounded with a semipermeable wall. The semipermeable wall comprises 95% cellulose acetate having an acetyl content of 39.8% and 5% poly(ethylene glycol) having a number average molecular weight of 3350. The semipermeable composition is dissolved in a solvent comprising acetone and water (90:10 wt:wt), to provide a solid concentration of 5%. The trilayer tablets are coated in a standard pan coater and the trilayer tablets are coated with a 12 mg wall. Next, a 30 mil (0.76 mm) orifice is drilled through the wall connecting the first drug layer with the exterior of the dosage form. The tablets are dried for 48 hours at 50°C and 50% relative humidity to remove the residual solvents.

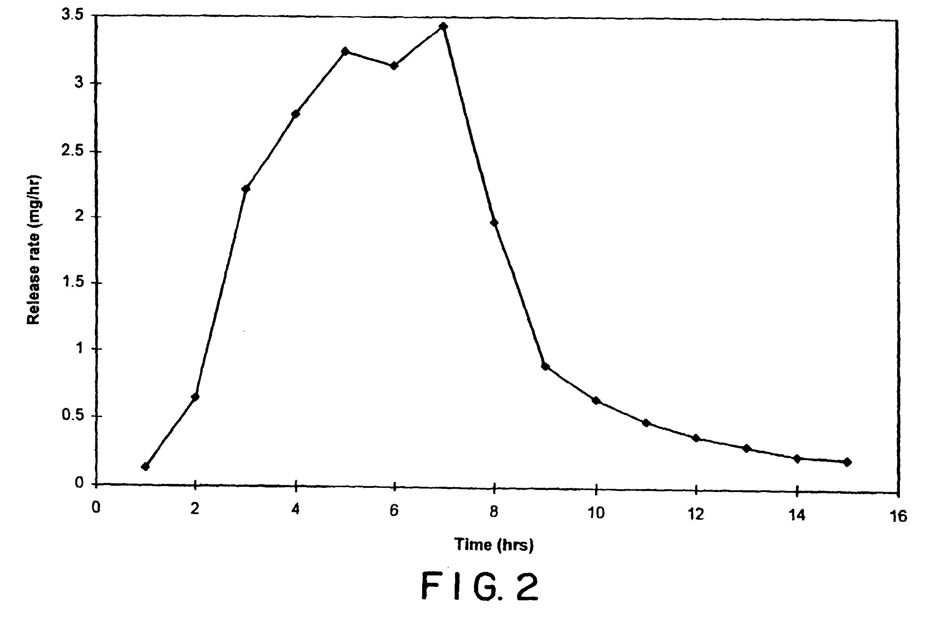

The dosage form comprises 4.4 mg of pseudoephedrine hydrochloride, 15.3 mg of poly(ethylene oxide) of number average molecular weight 300,000, 1.1 mg of hydroxypropylmethylcellulose of number average molecular weight 9,200, 1.1 mg of polyoxyethylene 40 stearate, and 0.11 mg of magnesium stearate in the first layer. The second layer comprises 13.5 mg of pseudoephedrine hydrochloride, 2.59 mg of poly(ethylene oxide) of number average molecular weight 300,000, 0.9 mg of hydroxypropylmethylcellulose of number average molecular weight 9,200, 0.9 mg of polyoxyethylene 40 stearate, 0.018 mg of red ferric oxide, and 0.09 mg of magnesium stearate. The third layer comprises 22.2 mg of poly(ethylene oxide) of number average molecular weight 7,000,000, 12 mg of sodium chloride, 2 mg of hydroxypropylmethylcellulose of number average molecular weight 9,200, 2 mg of polyoxyethylene 40 stearate, 1.2 mg of cross-linked acrylic acid polymer, 0.4 mg of red ferric oxide, and 0.2 mg of magnesium stearate. The semipermeable wall comprises 11.4 mg of cellulose acetate having a 39.8% acetyl content and 0.6 mg of polyethylene glycol of average number molecular weight 3350. The dosage form exhibited an ascending release-rate profile comprising 0.13 mg in 1 hour, 0.65 mg in 2 hours, 2.2 mg in 3 hours, 2.78 mg in 4 hours, 3.24 mg in 5 hours, 3.14 mg in 6 hours and 3.43 mg in 7 hours. The ascending release rate is seen in Figure 2.

### EXAMPLE 4

The dosage form prepared in this example follows the above example. In this example the displacement or third layer and the semipermeable wall are as prepared previously, accompanied by a new first layer and a new second layer.

The first drug layer is prepared as follows. First, 139 g of polyethylene oxide having a number average molecular weight of about 100,000 is passed through a 40 mesh screen and placed into the bowl of a conventional planetary mixer. Next, 40 g of pseudoephedrine hydrochloride is placed into the bowl containing the polyethylene oxide. Next, 10 g of hydroxypropylmethylcellulose of number average molecular weight 9,200 and 10 g of polyethylene 40 stearate is passed through a 40 mesh screen and placed into the bowl containing polyethylene oxide and pseudoephedrine hydrochloride. The four ingredients are blended together in the planetary mixer for 10 minutes. Next, 100 ml of denatured anhydrous ethanol is added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 1 g of magnesium stearate which has been passed through a 40 mesh screen is added to the granulation and all the ingredients are mixed for an additional 1 minute.

The second dry layer is prepared as follows: first, 28.8 g of polyethylene oxide having a number average molecular weight of about 200,000 is passed through a 40 mesh screen and placed into the bowl of a conventional planetary mixer. Next, 150 g of the drug pseudoephedrine hydrochloride is weighed and placed into the bowl containing the polyethylene oxide. Next, 10 g of hydroxypropylmethylcellulose of number average molecular weight 9,200, 10 g of polyoxyethylene 40 stearate, and 0.2 g of red ferric oxide are passed through a 40 mesh screen and placed into the bowl containing polyethylene oxide and pseudoephedrine hydrochloride. The composition is blended together in the planetary mixer for 10 minutes. Next, 100 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 1 g of magnesium stearate which has been passed through a 40 mesh screen is added to the granulation and all the ingredients are mixed for an additional 1 minute.

The dosage form prepared by this example comprised the following composition: a first drug layer comprising 20.0% pseudoephedrine hydrochloride, 69.5% polyethylene oxide of number average molecular weight 100,000, 5% hydroxypropyl methyl cellulose of number average molecular weight 9,200, 5% polyoxyethylene 40 stearate, and 0.5% magnesium stearate. The dosage form second drug layer comprising 75% pseudoephedrine hydrochloride, 14.4% polyethylene oxide of number average molecular weight 200,000, 5% hydroxypropyl methylcellulose of number molecular weight 9,200, 5% polyoxyethylene 40 stearate, 0.1% red ferric oxide, and 0.5% magnesium stearate. The third layer in the dosage form is composed of 55.5% polyethylene oxide of number average molecular weight 7,000,000, 30% sodium chloride, 5% hydroxypropylmethylcellulose, 5% polyoxyethylene 40 stearate, 3% cross linked acrylic acid polymer, 1 % red ferric oxide, and 0.5% magnesium stearate. The semipermeable wall comprises 95% cellulose acetate of acetyl content 39.8% and 5% polyethylene glycol having a number average molecular weight of 3350 applied to the compressed trilayer system, and a 30 mil orifice on the drug first layer as the exit orifice. The final system is capable of delivering 18 mg of pseudoephedrine hydrochloride with an ascending release rate over time.

### EXAMPLE 5

The dosage form prepared by this example follows the previous examples. In this example the third or displacement layer and the semipermeable wall are formulated as described previously, accompanied by a new first and new second layer.

The first drug layer is prepared as follows. First, 139 g of polyethylene oxide having a number average molecular weight of about 200,000 is passed through a 40 mesh screen and placed into the bowl of a conventional planetary mixer. Next, 40 g of the drug pseudoephedrine hydrochloride is weighed and placed into the bowl containing the polyethylene oxide. Next, 10 g of hydroxypropylmethylcellulose of number average molecular weight 9,200 and 10 g of polyoxyethylene 40 stearate (Myrj 52 S) is passed through a 40 mesh screen and placed into the bowl containing polyethylene oxide and pseudoephedrine hydrochloride. The four ingredients are blended together in the planetary mixer for 10 minutes. Next, 100 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 1 g of magnesium stearate which has been passed through a 40 mesh screen is added to the granulation and all the ingredients are mixed for an additional 1 minute.

The second drug layer is prepared as follows. First, 28.8 g of polyethylene oxide having a number average molecular weight of about 300,000 is passed through a 40 mesh screen and placed into the bowl of a conventional planetary mixer. Next, 150 g of the drug pseudoephedrine hydrochloride is weighed and placed into the bowl containing the polyethylene oxide. Next, 10 g of hydroxypropylmethylcellulose of number average molecular weight 9,200, 10 g of polyoxyethylene 40 stearate (Myrj 52 S) and 0.2 g of red ferric oxide are passed through a 40 mesh screen and placed into the bowl containing polyethylene oxide and pseudoephedrine hydrochloride. The composition is blended together in the planetary mixer for 10 minutes. Next, 100 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 1 g of magnesium stearate which has been passed through a 40 mesh screen is added to the granulation and all the ingredients are mixed for an additional 1 minute.

The dosage form prepared by this example comprised the following: a first drug layer comprising 20.0% pseudoephedrine hydrochloride, 69.5% polyethylene oxide of number average molecular weight 200,000, 5% hydroxypropylmethylcellulose of number average molecular weight 9,200, 5% polyoxyethylene 40 stearate, and 0.5% magnesium stearate. The second layer comprising 75.0% pseudoephedrine hydrochloride, 14.4% polyethylene oxide of number average molecular weight 300,000, 5% hydroxypropyl methyl cellulose of number average molecular weight 9,200, 5% polyoxyethylene 40 stearate, 0.1% red ferric oxide, and 0.5% magnesium stearate. The third layer in the dosage form is composed of 55.5% polyethylene oxide of number average molecular weight 7,000,000, 30% sodium chloride, 5% hydroxypropyl methyl cellulose of number average molecular weight 9,200, 5% polyoxyethylene 40 stearate (Myrj 52 S), 3% cross linked acrylic acid polymer (Carbomer 934P), 1% red ferric oxide and 0.5% magnesium stearate. 12 mg of a semipermeable wall comprised of 95% cellulose acetate of acetyl content 39.8% and 5% polyethylene glycol having a number average molecular weight of 3350 is coated to the compressed trilayer system and a 30 mil orifice is drilled on the drug layer side as the exit orifice. The final system is capable of delivering 18 mg of pseudoephedrine hydrochloride with an ascending release rate.

### EXAMPLES 6 & 7

The procedures disclosed above are followed to prepare the following dosage forms: (a) a dosage form wherein the first layer weighs 350 mg and comprises 12 wt% nicardipine, 52.80 wt% sorbitol, and 35.20 wt% poly(ethylene oxide) of number average molecular weight 200,000, a second 136 mg layer comprising 55 wt% nicardipine, 42 wt% poly(ethylene oxide) of number average molecular weight 300,000, and 3 wt% hydroxypropylmethylcellulose of number average molecular weight 9,200, a third 350 mg displacement layer comprising 68.75 wt% poly(ethylene oxide) of number average molecular weight 7,000,000, 20 wt% sodium chloride, 5 wt% hydroxypropylmethylcellulose of number average molecular weight 9,200, 1 wt% ferric oxide, 0.25 w% magnesium stearate, and 5 wt% acrylic acid polymer of number average molecular weight 3,000,000, a semipermeable wall comprising 90 wt% of a cellulose acetate of 39.8% acetyl content, and 10 wt% of polyethylene glycol of number average molecular weight 3350, a 25 mil (0.64 mm) orifice connecting the first layer with the exterior of the dosage form and wherein the dosage form has an ascending dose released over 16 hours; (b) a dosage form wherein the first layer comprises 350 mg consisting of 8.6 wt% nicardipine, 54.8 wt% sorbitol, and 36.80 poly(ethylene oxide) of number average molecular weight 200,000, a second drug layer weighing 120 mg comprising 45 wt% nicardipine, 50 wt% poly(ethylene oxide) of number average molecular weight 300,000, and 5 wt% of hydroxypropylmethylcellulose of number average molecular weight 11,200, a third displacement drug-free layer weighing 350 mg comprising 68.75 wt% poly(ethylene oxide) of number average molecular weight 7,000,000, 20 wt% sodium chloride, 0.25 wt% magnesium stearate lubricant, 5 wt% hydroxypropylmethylcellulose of number average molecular weight 9,200, 1 wt% ferric oxide and 5 wt% acrylic acid polymer of number average molecular weight 3,000,000, a wall weighing 43.50 mg comprising 95 wt% cellulose acetate of 39.8% acetyl content, and 5 wt% polyethylene glycol of number average molecular weight 3350, a 25 mil (0.64 mm) exit orifice and sustained continuous increasing dose over 16 hours. The dosage forms provided by these examples are characterized by a trilayer consisting of two drug layers and one displacement or push layer wherein the drug concentration of the second layer is larger than the drug concentration of the first layer, and the viscosity of the third layer is greater than the viscosity of the second layer and the viscosity of the second layer is greater than the viscosity of the first layer. Viscosity can be measured by standard techniques as in Pharmaceutical Sciences, by Remington, 17th Ed., pp. 342-345 (1985); and by using standard viscometers such as the Brookfield Viscometer, Model RVF, available from Brookfield Engineering Laboratories, Inc., Stoughton, Mass.

### EXAMPLE 8

An osmotic dosage form designed and shaped to deliver methylphenidate hydrochloride in an ascending release profile is manufactured as follows: 1) Composition of first drug layer: The following procedure is used to manufacture 200 g of the first drug layer composition: 157.8 of polyethylene oxide having a number average molecular weight of about 200,000 is passed through a 40 mesh screen and placed into the bowl of a conventional planetary mixer. Next, 31.2 g of the drug methylphenidate hydrochloride is weighed and placed into the bowl containing the polyethylene oxide. Next, 10 g of hydroxypropylmethylcellulose (HPMC-USP grade 2910 of number average molecular weight 9200) is passed through a 40 mesh screen and placed into the bowl containing polyethylene oxide and methylphenidate hydrochloride. Next, 0.5 g of FD&C Blue Dye #1 is placed into the bowl of the mixer. The four ingredients are blended together in the planetary mixer for 10 minutes. Next, about 100 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 0.5 g of magnesium stearate which has been passed through a 40 screen mesh is added to the granulation and all the ingredients are mixed for an additional minute. 2) Composition of the second layer: The following procedure is used to manufacture 200 g of the second drug layer composition: 112.6 g of polyethylene oxide having a number average molecular weight of about 200,000 is passed through a 40 mesh screen and placed into the bowl of a conventional planetary mixer. Next, 76.4 g of the drug methylphenidate hydrochloride is weighed and placed into the bowl containing the polyethylene oxide. Next, 10 g of hydroxypropylmethylcellulose (HPMC-USP grade 2910 of number average molecular weight 9200) is passed through a 40 mesh screen and placed into the bowl containing polyethylene oxide and methylphenidate hydrochloride. Next, 0.5 g of FD&C Blue Dye #1, or any other dye for color differentiation, is placed into the bowl of the mixer. The four ingredients are blended together in the planetary mixer for 10 minutes. Next, about 100 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours, and then passed through a 20 mesh screen. Next, 0.5 g of magnesium stearate which has been passed through a 40 mesh screen is added to the granulation and all the ingredients are mixed for an additional minute. 3) Composition of the third layer: The following procedure is used to manufacture 200 g of the third or push layer composition: 107 g of polyethylene oxide having a number average molecular weight of 7,000,000, 80 g of sodium chloride (40%), 10 g of hydroxypropylmethylcellulose (USP grade 2910 of number average molecular weight 9200) and 2 g of red ferric oxide are passed through a 40 mesh screen and then placed into the bowl of a conventional planetary mixer. The powder mixture is then blended together until a homogeneous blend is formed. Next, about 50 ml of denatured anhydrous ethanol is gradually added to the mixer with continued mixing over a period of 5 to 10 minutes such that the consistency of the dry powder changes to that of granules. The wet granulation is then passed through a 20 mesh screen, dried at room temperature for 16 hours and then passed through a 20 mesh screen. Next, 1 g of magnesium stearate which has been passed through a 40 mesh is added to the granulation and all the ingredients are mixed for an additional 1 minute.

A layer press is used to compress the two layers together to form a tablet dosage form. First, 33 mg of layer 1 is added to the 7/32" (0.55 cm) die cavity and lightly tamped. Next, 24 mg of layer 2 is weighed and placed into the die cavity and lightly tamped. Next, 57 mg of layer 3 is added to the die cavity and the three layers are compressed together using about 1/2 ton of pressure to form a trilayer tablet.

The semipermeable membrane composition that provides the wall of the dosage form is composed of 95% cellulose acetate (having an acetyl content of 39.8%) and 5% polyethylene glycol having a number average molecular weight of 3350. The semipermeable membrane composition is dissolved in a mixture of acetone and water (the solvents are mixed together in a ratio of 90:10 wt:wt), such that the solids composition of the solution is 5%. The trilayer tablets are placed in a pan coater and about 15 mg of the semipermeable membrane composition is sprayed onto the trilayer tablets. Next, a 30 mil (0.76 mm) orifice is drilled on the drug layer side of the tablet using a mechanical drill. Next, the tablets are dried for 48 hours at 50°C and 50% relative humidity to remove the residual solvents. Next, an application of drug containing overcoat and a taste masking coat is overcoated onto the exterior surface of the wall. The drug containing overcoat is composed of 60% hydroxypropylmethylcellulose of average molecular weight 9,200 and 40% methylphenidate hydrochloride. The hydroxypropylmethylcellulose is added to water and mixed until a uniform solution results. Then, the methylphenidate hydrochloride is added to this solution and mixed such that a clear solution results. The final solution has a solids composition of 10%. The membrane coated systems are placed in a coater and 10 mg of the drug overcoat solution is sprayed on to the bilayer tablets. Next, the tablets are dried in the coating pan at 40°C for 10-15 minutes. For the taste masking coat, a suspension of Opadry® (a commercially obtained product from Colorcon, which is a film forming concentrate composed of hydroxypropylmethylcellulose, titanium dioxide, polyethylene glycol, polysorbate 80 and a dye for product identification) is prepared in water such that the solid content is 10%. The systems coated with the drug containing overcoat are placed into the coater and the 9 mg of taste masking solution is sprayed onto the systems. Next, the systems are dried in the coating pan at 40°C for 10-15 minutes.

A methylphenidate dosage form as described in this example, contains 33 mg of drug containing drug layer 1 which is composed of 15.6% methylphenidate hydrochloride, 78.9% polyethylene oxide of number average molecular weight 200,000, 5% hydroxypropylmethylcellulose (USP-2910 of number average molecular weight 9200), 0.25% magnesium stearate and 0.25% FD&C Blue Dye #1. The dosage form also contains 24 mg of drug layer 2 composed of 38.2% methylphenidate hydrochloride, 56.32% polyethylene oxide of number average molecular weight 200,000, 5% hydroxypropylmethylcellulose (USP-2910 of number average molecular weight 9200), 0.25% magnesium stearate, and 0.25% FD&C blue dye #1 or any other dye. The third layer in the dosage form is composed of 53.5% polyethylene oxide of number average molecular weight 7,000,000, 40% sodium chloride, 5% hydroxypropylmethylcellulose, 1% red ferric oxide, and 0.5% magnesium stearate. 15 mg of a semipermeable laminate composed of 95% cellulose acetate of acetyl content 39.8% and 5% polyethylene glycol having a number average molecular weight of 3350 is applied to the compressed trilayer system and a 30 mil orifice is drilled on the drug layer side as the exit orifice. 10 mg of a drug overcoat composed of 6 mg hydroxypropylcellulose and 4 mg methylphenidate hydrochloride is applied to the membrane coated system. 9 mg of a final taste masking coat composed of 100% Opadry® is applied. The final system is capable of delivering 18 mg of methylphenidate hydrochloride where 4 mg are released immediately from the overcoat and 14 mg is released with an ascending release rate from the interior of the dosage form.

### DISCLOSURE OF METHOD OF USING THE INVENTION

The invention pertains further to a method for delivering an ascending dose over time, to a warm-blooded animal in need of therapy. The method comprises the steps of: (A) admitting into a patient a dosage form comprising: (1) a wall that surrounds a compartment, the wall comprises a semipermeable composition permeable to the passage of fluid, including aqueous-biological fluid of the gastrointestinal tract, and impermeable to the passage of drug; (2) a trilayer in the compartment comprising a first layer comprising a dose of drug, a second layer comprising a larger dose of drug, and a third layer comprising an osmotic formulation for imbibing and absorbing fluid, and for pushing the first and second layers from the dosage form, thereby providing an increased dose per unit time over time; and (3) at least one exit in the wall communicating with the first layer; (B) imbibing fluid through the semipermeable wall at a rate determined by the permeability of the semipermeable wall and the osmotic gradient across the semipermeable wall causing the third layer to expand and swell; and (C) deliver the drug from the first layer followed by delivering the drug from the second layer through the exit passageway to provide an ascending increasing dose of drug to the patient.

In summary, it will be appreciated the present invention contributes to the art an unexpected dosage form that possesses the practical utility for administering a sustained and increasing dose of drug at a dosage metered release rate over time.

## Claims

1. A pharmaceutical tablet comprising:-
(a) a first layer comprising 10 ng to 300 mg dose of a drug and 5 mg to 250 mg of a hydrophilic polymer; and
(b) a second layer comprising 50 ng to 500 mg dose of a drug and 25 mg to 450 mg of a hydrophilic polymer and wherein the tablet comprises a larger dose of drug in the second layer and drug is released from the tablet at a rate per unit time that ascends continuously for a sustained period of time.

2. The tablet according to claim 1 wherein the tablet comprises a larger dose of the same drug in the second layer than in the first layer.

3. The tablet according to claim 1 or claim 2 wherein the drug is selected from a central nervous system stimulant, a stimulant, a cannabinoid and a catecholamine drug.

4. The tablet according to any preceding claim wherein the drug in the first layer and in the second layer comprises a member selected from amphetamine dextro-amphetamine, methamphetamine, methylphenidate, racemic methylphenidate, theomethylphenidate, ethylphenidate, phenylisoproopylamine, and pemoline.

5. The tablet of any of claims 1 to 4 comprising:-
(c) a third layer comprising 15 ng to 450 mg of a hydrophilic polymer which third layer provides support for the first and second layer to deliver the drug consecutively therefrom.

6. The tablet according to claim 5 wherein the first and second layers comprise the same drug and the third layer is free of drug.

7. The tablet according to claim 5 or 6, wherein the first and second layers comprise a poly(alkylene oxide) of 40,000 to 1,000,000 average molecular weight and the third layer comprises a poly(alkylene oxide) of 2,000,000 to 10,000,000 average molecular weight.

8. The tablet according to claim 7 wherein the first and second layers further comprise a hydroxypropylemethylcellulose of 9,000 to 750,000 average molecular weight.

9. A dosage form comprising:-
(a) a first layer comprising 10 ng to 300 mg of a drug,
(b) a second layer comprising 50 ng to 500 mg of a drug which second layer comprises more drug than the first layer;
(c) a third layer for displacing the first layer followed by the second layer from the dosage form;
(d) a wall that surrounds the three layers; and
(e) a passageway in the wall communicating with the first drug layer for delivering the first drug layer followed by the second drug layer from the dosage form whereby drug is released from the dosage form at a rate per unit time that ascends continuously for a sustained period of time.

10. The dosage form according to claim 9 wherein the drug in the first and second layers is the same drug selected from a central nervous system stimulant, a stimulant and a catecholamine drug.

11. The dosage form according to either claims 9 or 10 wherein the three layers comprise a poly(alkylene oxide).

12. The dosage form according to either of claims 9 or 10 wherein the first and second layers comprise a hydroxypropylalkylcellulose, a carboxymethylcellulose, or a carboxyvinylpolymer.

13. The dosage form according to either of claims 9 or 10 wherein the first and second layers comprise a hydroxyalkylcellulose.

14. The dosage form of either of claims 9 or 10 wherein the third layer comprises a carboxymethylcellulose or carboxyvinylpolymer.

15. The dosage form according to any one of claims 9 to 14 wherein the drug of the first layer is methylphenidate;
the drug of the second layer is methylphenidate, which second layer comprises a larger dose of methylphenidate than the first layer; and
wherein the wall that surrounds the three layers is permeable to fluid and impermeable to methylphenidate.

16. The dosage form according to claim 15 wherein the first layer comprises 1 mg to 250 mg to poly(ethylene oxide) and/or wherein the second layer comprises 1 mg to 450 mg of poly(ethylene oxide).

17. The dosage form according to claim 15 wherein at least one of the first and second layers comprises at least one component selected from:-
0.5 to 7.5 mg of a surfactant;
0.5 to 20 mg of hydroxypropylmethylcellulose;
up to 20 mg of hydroxypropylcellulose;
up to 100 mg of a carboxyvinylpolymer; and
up to 250 mg of carboxymethylcellulose.

18. The dosage form according to any of claims 15 to 17 wherein the third layer comprises at least one component selected from a poly(ethylene oxide) of 2,000,000 to 10,000,000 average molecular weight;
a carboxymethylcellulose of 2,000,000 to 10,000,000 average molecular weight;
a carboxyvinylpolymer of 750,000 to 10,000,000 molecular weight; and
a hydroxypropylalkylcellulose of 9,200 to 750,000 average molecular weight.

19. A dosage form tablet for oral administration of methylphenidate wherein the dosage form tablet comprises:-
(a) a first layer comprising 10 ng to 350 mg of methylphenidate;
(b) a second layer comprising 10 ng to 500 mg of methylphenidate;
(c) a third layer comprising 15 ng to 450 mg of a hydrophilic polymer;
(d) a wall comprising a semipermeable composition that surrounds the first, second and third layers;
(e) a passageway in the wall communicating with the first layer for delivering the first layer followed by the second layer from the dosage form tablet whereby drug is released from the dosage form tablet at a rate per unit time that ascends continually for a sustained period of time; and
(f) an overcoat comprising 10 ng to 20 mg of methylphenidate on the exterior surface of the wall.

## Patentansprüche

1. Pharmazeutische Tablette, umfassend:
(a) eine erste Schicht, die 10 ng bis 300 mg einer Dosis eines Medikaments und 5 mg bis 250 mg eines hydrophilen Polymers umfaßt; und
(b) eine zweite Schicht, die 50 ng bis 500 mg einer Dosis eines Medikaments und 25 mg bis 450 mg eines hydrophilen Polymers umfaßt, wobei die Tablette eine höhere Dosis des Medikaments in der zweiten Schicht umfaßt, und das Medikament aus der Tablette mit einer Rate pro Zeiteinheit freigesetzt wird, die kontinuierlich über einen verzögerten Zeitraum ansteigt.

2. Tablette nach Anspruch 1, worin die Tablette in der zweiten Schicht eine höhere Dosis des gleichen Medikaments als in der ersten Schicht umfaßt.

3. Tablette nach Anspruch 1 oder Anspruch 2, worin das Medikament ausgewählt ist aus einem Stimulans des zentralen Nervensystems, einem Stimulans, einem Cannabinoid und einem Katecholamin-Medikament.

4. Tablette nach einem der vorhergehenden Ansprüche, worin das Medikament in der ersten Schicht und in der zweiten Schicht ein Mitglied umfaßt, das ausgewählt ist aus Amphetamin, Dextroamphetamin, Methamphetamin, Methylphenidat, racemischem Methylphenidat, Theomethylphenidat, Ethylphenidat, Phenylisopropylamin und Pemolin.

5. Tablette nach einem der Ansprüche 1 bis 4, umfassend:
(c) eine dritte Schicht, die 15 ng bis 450 mg eines hydrophilen Polymers umfaßt, wobei die dritte Schicht die erste und zweite Schicht dabei unterstützt, das Medikament fortlaufend daraus abzugeben.

6. Tablette nach Anspruch 5, worin die erste und zweite Schicht das gleiche Medikament umfassen, und die dritte Schicht medikamentenfrei ist.

7. Tablette nach Anspruch 5 oder 6, worin die erste und zweite Schicht ein Poly(alkylenoxid) mit einer durchschnittlichen Molmasse von 40 000 bis 1 000 000 umfassen, und die dritte Schicht ein Poly(alkylenoxid) mit einer durchschnittlichen Molmasse von 2 000 000 bis 10 000 000 umfaßt.

8. Tablette nach Anspruch 7, worin die erste und zweite Schicht weiterhin eine Hydroxypropylmethylcellulose mit einer durchschnittlichen Molmasse von 9 000 bis 750 000 umfassen.

9. Dosierungsform, umfassend:
(a) eine erste Schicht, die 10 ng bis 300 mg eines Medikaments umfaßt,
(b) eine zweite Schicht, die 50 ng bis 500 mg eines Medikaments umfaßt, wobei die zweite Schicht mehr Medikament als die erste Schicht umfaßt;
(c ) eine dritte Schicht, die die erste Schicht und dann die zweite Schicht aus der Dosierungsform verdrängt;
(d) eine Wand, welche die drei Schichten umgibt, und
(e) einen Durchgang in der Wand, der mit der ersten Medikamentenschicht verbunden ist, um die erste Medikamentenschicht und dann die zweite Medikamentenschicht aus der Dosierungsform abzugeben, wobei das Medikament aus der Dosierungsform mit einer Rate pro Zeiteinheit freigesetzt wird, die kontinuierlich über einen verlängerten Zeitraum ansteigt.

10. Dosierungsform nach Anspruch 9, worin das Medikament in der ersten und zweiten Schicht das gleiche Medikament ist, das ausgewählt ist aus einem Stimulans des zentralen Nervensystems, einem Stimulans und einem Katecholamin-Medikament.

11. Dosierungsform nach einem der Ansprüche 9 oder 10, worin die drei Schichten ein Poly(alkylenoxid) umfassen.

12. Dosierungsform nach einem der Ansprüche 9 oder 10, worin die erste und die zweite Schicht eine Hydroxypropylalkylcellulose, eine Carboxymethylcellulose oder ein Carboxyvinylpolymer umfassen.

13. Dosierungsform nach einem der Ansprüche 9 oder 10, worin die erste und zweite Schicht eine Hydroxyalkylcellulose umfassen.

14. Dosierungsform nach einem der Ansprüche 9 oder 10, worin die dritte Schicht eine Carboxymethylcellulose oder ein Carboxyvinylpolymer umfaßt.

15. Dosierungsform nach einem der Ansprüche 9 bis 14, worin das Medikament der ersten Schicht Methylphenidat ist;
das Medikament der zweiten Schicht Methylphenidat ist, wobei die zweite Schicht eine höhere Dosis an Methylphenidat als die erste Schicht umfaßt; und
worin die Wand, welche die drei Schichten umgibt, gegenüber Flüssigkeit durchlässig und gegenüber Methylphenidat undurchlässig ist.

16. Dosierungsform nach Anspruch 15, worin die erste Schicht 1 mg bis 250 mg Poly(ethylenoxid) umfaßt und/oder worin die zweite Schicht 1 mg bis 450 mg Poly(ethylenoxid) umfaßt.

17. Dosierungsform nach Anspruch 15, worin mindestens eine der ersten und zweiten Schicht mindestens eine Komponente umfaßt, die ausgewählt ist aus:
0,5 bis 7,5 mg eines oberflächenaktiven Mittels;
0,5 bis 20 mg Hydroxypropylmethylcellulose;
bis zu 20 mg Hydroxypropylcellulose;
bis zu 100 mg eines Carboxyvinylpolymers;
bis zu 250 mg Carboxymethylcellulose.

18. Dosierungsform nach einem der Ansprüche 15 bis 17, worin die dritte Schicht mindestens eine Komponente umfaßt, die ausgewählt ist aus einem Poly(ethylenoxid) mit einer durchschnittlichen Molmasse von 2 000 000 bis 10 000 000;
einer Carboxymethylcellulose mit einer durchschnittlichen Molmasse von 2 000 000 bis 10 000 000 ;
einem Carboxyvinylpolymer mit einer Molmasse von 750 000 bis 10 000 000;
und einer Hydroxypropylalkylcellulose mit einer durchschnittlichen Molmasse von 9 200 bis 750 000.

19. Dosierungsformtablette zur oralen Verabreichung von Methylphenidat, worin die Dosierungsformtablette umfaßt:
(a) eine erste Schicht, die 10 ng bis 350 mg Methylphenidat umfaßt;
(b) eine zweite Schicht, die 10 ng bis 500 mg Methylphenidat umfaßt;
(c) eine dritte Schicht, die 15 ng bis 450 mg eines hydrophilen Polymers umfaßt;
(d) eine Wand, die eine semipermeable Zusammensetzung umfaßt, welche die erste, zweite und dritte Schicht umgibt;
(e) einen Durchgang in der Wand, der mit der ersten Schicht verbunden ist, um die erste Schicht und dann die zweite Schicht aus der Dosierungsformtablette abzugeben, wobei das Medikament aus der Dosierungsformtablette mit einer Rate pro Zeiteinheit freigesetzt wird, die kontinuierlich über einen verlängerten Zeitraum ansteigt; und
(f) einen Überzug, der 10 ng bis 20 mg Methylphenidat auf der Außenfläche der Wand umfaßt.

## Revendications

1. Comprimé pharmaceutique comprenant :
(a) une première couche comprenant une dose de 10 ng à 300 mg d'un médicament et 5 mg à 250 mg d'un polymère hydrophile ; et
(b) une deuxième couche comprenant une dose de 50 ng à 500 mg d'un médicament et 25 mg à 450 mg d'un polymère hydrophile, le comprimé comprenant une dose de médicament plus grande dans la deuxième couche et le médicament étant libéré du comprimé dans une quantité par unité de temps qui augmente de façon continue pendant une période de temps prolongée.

2. Comprimé selon la revendication 1, dans lequel le comprimé comprend une plus grande dose du même médicament dans la deuxième couche que dans la première couche.

3. Comprimé selon la revendication 1 ou la revendication 2, dans lequel le médicament est choisi parmi un stimulant du système nerveux central, un stimulant, un cannabinoïde et un médicament à base de catécholamine.

4. Comprimé selon l'une quelconque des revendications précédentes, dans lequel le médicament dans la première couche et dans la deuxième couche comprend un élément choisi parmi l'amphétamine, la dextro-amphétamine, la méthamphétamine, le méthylphénidate, le méthylphénidate racémique, le théométhylphénidate, l'éthylphénidate, la phénylisopropylamine et la pémoline.

5. Comprimé selon l'une quelconque des revendications 1 à 4, comprenant :
(c) une troisième couche comprenant 15 ng à 450 mg d'un polymère hydrophile, laquelle troisième couche fournit un support pour que les première et deuxième couches administrent le médicament de façon consécutive à partir de celles-ci.

6. Comprimé selon la revendication 5, dans lequel les première et deuxième couches comprennent le même médicament et la troisième couche est exempte de médicament.

7. Comprimé selon l'une des revendications 5 ou 6, dans lequel les première et deuxième couches comprennent un poly(oxyde d'alkylène) d'une masse moléculaire moyenne de 40 000 à 1 000 000, et la troisième couche comprend un poly(oxyde d'alkylène) d'une masse moléculaire moyenne de 2 000 000 à 10 000 000.

8. Comprimé selon la revendication 7, dans lequel les première et deuxième couches comprennent en outre une hydroxypropylméthyl cellulose d'une masse moléculaire moyenne de 9000 à 750 000.

9. Forme posologique comprenant :
(a) une première couche comprenant 10 ng à 300 mg d'un médicament ;
(b) une deuxième couche comprenant 50 ng à 500 mg d'un médicament, laquelle deuxième couche comprend davantage de médicament que la première couche ;
(c) une troisième couche pour déplacer la première couche, suivie par la deuxième couche à partir de la forme posologique ;
(d) une paroi qui entoure les trois couches ; et
(e) un passage dans la paroi communiquant avec la première couche de médicament pour administrer la première couche de médicament suivie par la deuxième couche de médicament à partir de la forme posologique, ce par quoi le médicament est libéré de la forme posologique dans une quantité par unité de temps qui augmente de façon continue pendant une période de temps prolongée.

10. Forme posologique selon la revendication 9, dans laquelle le médicament dans les première et deuxième couches est le même médicament choisi parmi un stimulant du système nerveux central, un stimulant et un médicament à base de catécholamine.

11. Forme posologique selon l'une des revendications 9 ou 10, dans laquelle les trois couches comprennent un poly(oxyde d'alkylène).

12. Forme posologique selon l'une des revendications 9 ou 10, dans laquelle les première et deuxième couches comprennent une hydroxypropylalkylcellulose, une carboxyméthylcellulose ou un polymère carboxyvinylique.

13. Forme posologique selon l'une des revendications 9 ou 10, dans laquelle les première et deuxième couches comprennent une hydroxyalkylcellulose.

14. Forme posologique selon l'une des revendications 9 ou 10, dans laquelle la troisième couche comprend une carboxyméthylcellulose ou un polymère carboxyvinylique.

15. Forme posologique selon l'une des revendications 9 à 14, dans laquelle :
- le médicament de la première couche est le méthylphénidate ;
- le médicament de la deuxième couche est le méthylphénidate, laquelle deuxième couche comprend une dose plus grande de méthylphénidate que la première couche ; et
dans laquelle la paroi qui entoure les trois couches est perméable au fluide et imperméable au méthylphénidate.

16. Forme posologique selon la revendication 15, dans laquelle la première couche comprend 1 mg à 250 mg de poly(oxyde d'éthylène) et/ou dans laquelle la deuxième couche comprend 1 mg à 450 mg de poly(oxyde d'éthylène).

17. Forme posologique selon la revendication 15, dans laquelle au moins l'une des première et deuxième couches comprend un composant choisi parmi :
- 0,5 à 7,5 mg d'un agent tensio-actif ;
- 0,5 à 20 mg d'hydroxypropylméthylcellulose ;
- jusqu'à 20 mg d'hydroxypropylcellulose ;
- jusqu'à 100 mg de polymère carboxyvinylique ; et
- jusqu'à 250 mg de carboxyméthylcellulose.

18. Forme posologique selon l'une quelconque des revendications 15 à 17, dans laquelle la troisième couche comprend au moins un composant choisi parmi :
- un poly(oxyde d'éthylène) d'une masse moléculaire moyenne de 2 000 000 à 10 000 000 ;
- une carboxyméthylcellulose d'une masse moléculaire moyenne de 2 000 000 à 10 000 000 ;
- un polymère carboxyvinylique d'une masse moléculaire de 750 000 à 10 000 000 ; et
- une hydroxypropylalkylcellulose d'une masse moléculaire moyenne de 9 200 à 750 000.

19. Comprimé de forme posologique pour une administration orale de méthylphénidate dans lequel le comprimé de forme posologique comprend :
(a) une première couche comprenant 10 ng à 350 mg de méthylphénidate ;
(b) une deuxième couche comprenant 10 ng à 500 mg de méthylphénidate ;
(c) une troisième couche comprenant 15 ng à 450 mg d'un polymère hydrophile ;
(d) une paroi comprenant une composition semiperméable qui entoure les première, deuxième et troisième couches ;
(e) un passage dans la paroi communiquant avec la première couche pour administrer la première couche suivie par la deuxième couche à partir du comprimé de forme posologique, ce par quoi le médicament est libéré du comprimé de forme posologique dans une quantité par unité de temps qui augmente de façon continue pendant une période de temps prolongée ; et
(f) un revêtement supérieur comprenant 10 ng à 20 mg de méthylphénidate sur la surface extérieure de la paroi.
